# FASCICULE DE BREVET EUROPEEN

(11) **EP 0 602 159 B1**
(45) Date de publication et mention de la délivrance du brevet: **04.12.1996**
(21) Numéro de dépôt: 92919482.7
(22) Date de dépôt: 04.09.1992
(51) Int. Cl.: A61F 2/42, A61L 27/00

(54) **PROTHESE ARTICULAIRE DIGITALE POUR ARTICULATIONS METACARPOPHALANGIENNES ET INTERPHALANGIENNES**
FINGERGELENKPROTHESE FÜR METAKARPALE UND INTERPHALANGÄRE GELENKE
FINGER JOINT PROSTHESIS FOR METACARPALPHALANGEAL AND INTERPHALANGEAL JOINTS

(30) Priorité: 06.09.1991 FR 9111043
(43) Date de publication de la demande: 22.06.1994
(73) Titulaire: COMMISSARIAT A L'ENERGIE ATOMIQUE, 75015 Paris Cédex 15 (FR)
(72) Inventeur: BROCHIER, Michel, F-38560 Jarrie (FR); GAIDRY, Jacques, F-38320 Herbeys (FR); MOREAU, Claude, F-38250 S.-Nizier (FR); MOUTET, François, F-38240 Meylan (FR); RANC, René, F-38100 Grenoble (FR)
(74) Mandataire: Dubois-Chabert, Guy
(86) Numéro de dépôt international: FR9200845
(87) Numéro de publication internationale: WO9304644

(56) Documents cités:
- EP-A- 0 055 406
- EP-A- 0 214 773
- WO-A-90/11739
- FR-A- 2 566 272
- FR-A- 2 590 794
- GB-A- 1 328 497
- GB-A- 2 015 881
- GB-A- 2 015 882
- US-A- 3 813 700

## Description

La présente invention se rapporte au domaine des prothèses articulaires digitales utilisées pour remplacer les articulations phalangiennes et métacarpophalangiennes.

Diverses maladies en effet, telles que l'arthrose post-traumatique, la polyarthrite évolutive, l'arthrite septique et certains états rhumatismaux conduisent à la détérioration progressive, voire à la destruction des articulations digitales. C'est pourquoi, depuis un certain nombre d'années, des prothèses ou implants articulaires de diverses natures ont été mis au point dans le domaine chirurgical pour tenter de restaurer l'usage des doigts de la main chez les patients atteints de telles maladies.

La destruction des articulations interphalangiennes proximales (IPP) et métacarpophalangiennes (MP) des doigts longs, pose au chirurgien de la main un problème thérapeutique encore incomplètement résolu _{|}(1) PELLEGRINI V.D., BURTON R.I., Osteoarthritis of the proximal incerphalangeal joint of the hand, arthroplasty or fusion, J. Hand Surg. 1990, 15-A,2,194-209_{|}. La récupération d'amplitudes fonctionnelles nécessite le remplacement des surfaces articulaires, que la destruction articulaire soit la conséquence d'une arthrose post-traumatique, de l'évolution d'une polyarthrite évolutive ou d'une arthrite septique.

On commencera d'abord par rappeler les principales solutions envisagées et appliquées à ce jour en indiquant à chaque fois leurs inconvénients.

L'apparition des implants en silicone _{|}(2) SWANSON A.B. Silicone rubber implants for replacement of arthritic or destroyed joints in the hand. Surg. Clin. North Am., 1968, 48,1113-1127_{|}, a marqué un progrès certain dans ce domaine. Mais avec le temps sont apparues nombre de complications propres à ce matériel telles que : ruptures d'implants, instabilités latérales, manque de force, douleurs résiduelles. Les réactions aux corps étrangers en silicone, source possible de pathologie auto-immune rendent leur emploi aléatoire surtout chez le sujet jeune. _{|}(3) EKFORS O. ARO H. MAKI J. AHO A.J. Cystic osteolysis induced by silicone rubber prosthesis. Arch. Pathol. Lab. Med. 1984, 108, 225-227_{|}.

Les résultats décevants de ce type d'implant, surtout en post-traumatique ont fait rechercher d'autres solutions arthroplastiques _{|}(4) BLAIR W.F., SHURR D.G., BUCKWALTER J.A. Metacarpophalangeal joint arthroplasty with a metallic hinged prosthesis. Clin. Orthop. 1984, 184, 156-163_{|}, et _{|}(5) CUNDAMINE J.L., BENOIT J.Y., COMTET J.J., AUBRIOT J. Proposition pour une arthroplastie digitale. Etude critique des premiers résultats. Ann. Chir. Main, 1988,7,4, 282-297_{|}.

Deux concepts d'arthroplastie sont possibles : les arthroplasties contraintes d'une part, où les deux parties articulées sont mécaniquement liées ; les semi et non-contraintes de l'autre où les deux pièces sont sans liaison mécanique.

Les problèmes de stabilité latérale que rencontrent les prothèses non, ou semi-contraintes (5) ont conduit le demandeur à opter pour un implant contraint à charnière ne possédant donc qu'un degré de liberté, celui de la flexion-extension.

Ce type d'implant à charnière soulève deux problèmes : celui de la résistance et de la fiabilité de la charnière elle-même (4),(5), et celui de l'ancrage des pièces diaphysaires qu'elles soient scellées ou non (5).

Les arthroplasties existantes sont réalisées outre le silicone, en acier inoxydable et polyéthylène (5) ou en titane et polyéthylène (4).

Le document GB-A-2 015 881 divulgue une articulation pour prothèse articulaire comprenant une première pièce destinée à être fixée à un premier os et ayant une surface d'appui courbe à une extrémité, une seconde pièce destinée à être fixée à un second os et ayant une chape femelle à une extrémité, et une troisième pièce ayant une surface d'appui courbe, les surfaces d'appui courbes des première et troisième pièces étant conjugées entre elles. Dans cette articulation, la troisième pièce est disposée dans la chape femelle de la seconde pièce. Un axe assure le maintien de l'ensemble.

On peut enfin citer comme illustration de l'état de la technique, les documents FR-A-2 590 794, FR-A-2 605 878 et FR-A-2 620 932. Dans ces trois documents on trouve divulguée l'utilisation de pièces diaphysaires ou broches destinées à être fixées dans le canal médullaire des os des doigts, ces pièces diaphysaires débouchant à leur extrêmité commune sur un système de rotation et/ou d'emboîtement rotulant possédant 1 ou 2 degrés de liberté et qui permettent de reconstituer une liberté de mouvement digital aussi proche que possible de celle que procurent les articulations naturelles.

La présente invention a précisément pour objet une prothèse articulaire digitale pour les articulations métacarpophalangiennes ou interphalangiennes qui permet de s'affranchir des inconvénients précédemment rappelés de l'art antérieur en associant dans une charnière de conception originale, les matériaux que sont le titane et ses alliages d'une part, et le carbone pyrolytique.

Cette prothèse articulaire digitale, implantable dans les os digitaux, comprend les caractéristiques du préambule de la revendication 1 et est caractérisée par les caractéristiques de la partie caractérisante de la revendication 1.

Dans une réalisation préférentielle, les chapes mâle et femelle ainsi que les flasques latéraux sont en alliage de titane, et la bague, l'axe et les joues sont en pyrocarbone de façon à ce que les frottements lors de la rotation de l'articulation aient lieu uniquement entre des pièces en pyrocarbone.

Le champ des applications potentielles de la prothèse articulaire, objet de l'invention, est très large et intéresse pratiquement tous les cas de destruction articulaire définitive des articulations de type MP et de type IPP quelles qu'en soit l'origine.

La charnière proprement dite de la prothèse est constituée de deux pièces médullaires en alliage de titane (TA6V) réunies par un axe en pyrocarbone, libre en rotation dans un palier également en pyrocarbone. Le pyrocarbone est obtenu à l'état de dépôt sur un substrat compatible (généralement le graphite) par décomposition thermique d'un hydrocarbure gazeux. L'axe de l'articulation est fabriqué selon cette technique par revêtement d'un cylindre de graphite. Selon la revendication 2, la structure de la prothèse permet d'éviter tout contact ou frottement autre que pyrocarbone sur pyrocarbone. Le faible coefficient de frottement et la grande résistance à l'usure d'un tel couple largement vérifiés sur banc d'essai pour cette Structure particulière, associés à la biocompatibilité universellement reconnue du pyrocarbone confèrent une excellente fiabilité et une très bonne tolérance à cette prothèse. Le titane quant à lui est garant de la rigidité et de la solidité des tiges médullaires. Sa biocompatibilité et ses qualités mécaniques sont bien connues de l'homme du métier. Un couple de matériaux de grande robustesse et de grande fiabilité dans le temps est donc ainsi réuni dans l'association du titane et du pyrocarbone.

La charnière ainsi réalisée a un débattement de 120° en extension comme en flexion. En cas de nécessité, par exemple, baisse du tonus de l'appareil fléchisseur, il est possible de créer un frein à l'extension passive de la charnière, par mise en place d'une mini-outée.

Enfin, la fixation des tiges médullaires est effectuée au moyen de métacrylate de méthyle afin de permettre une mobilisation active sans limitation en phase post-opératoire immédiate.

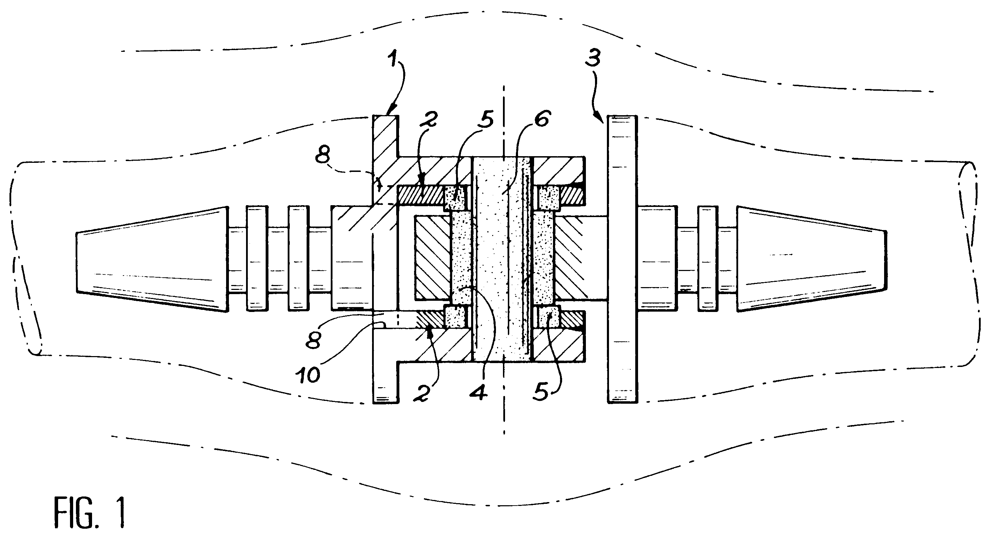

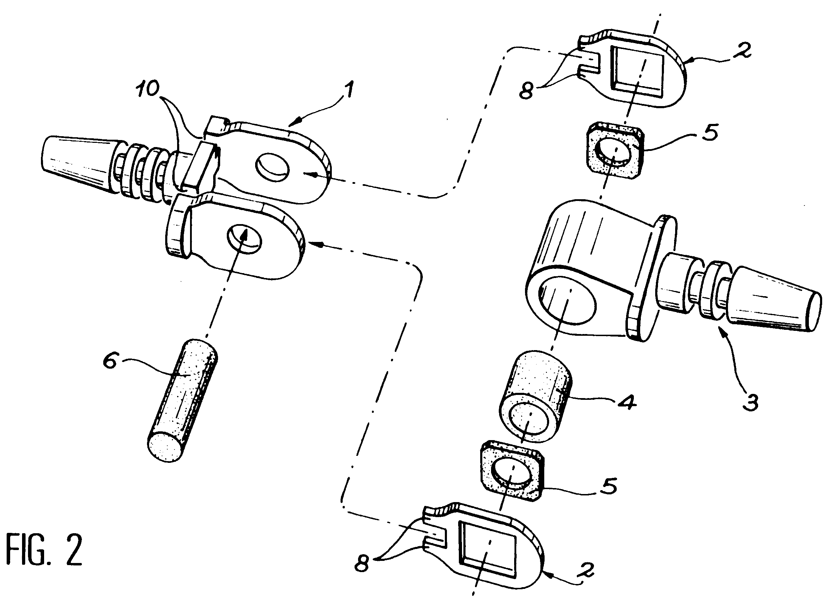

L'invention sera mieux comprise en se référant à la description qui suit d'un exemple de réalisation de la prothèse articulaire digitale, exemple qui sera décrit de façon schématique, à titre illustratif et non limitatif en se référant aux figures 1 et 2 ci-jointes sur lesquelles :
- la figure 1 est une vue en coupe transversale d'une prothèse articulaire digitale conforme à l'invention ;
- la figure 2 représente en vue éclatée les différents composants de cette prothèse articulaire.

Sur la figure 1, on voit les deux pièces diaphysaires 1 et 3 munies de façon classique de gorges destinées à faciliter leur fixation dans le canal médullaire des phalanges ou des métacarpes dans lesquelles elles sont introduites. La pièce diaphysaire ou broche 1 se termine à son extrémité située du côté de l'articulation par une chape femelle munie de deux orifices alignés et transverses par rapport à l'axe de la broche. Ces derniers détails seront vus plus clairement sur la vue éclatée de la figure 2. La pièce diaphysaire 3, opposée à la pièce 1, se termine par une chape mâle également mieux visible sur la figure 2 et qui sera décrite par conséquent ultérieurement. Cette chape mâle est munie d'un orifice transverse par rapport à la pièce diaphysaire 3 qui est destinée à coopérer avec la chape femelle de la pièce diaphysaire 1. Un axe 6 est monté à force dans les deux orifices de la chape femelle et à glissement doux dans la bague 4 montée en force dans l'orifice de la chape mâle ; il constitue à proprement parler l'axe de rotation de la prothèse. Une bague 4 entoure l'axe 6 et elle est engagée dans l'orifice de la chape mâle servant ainsi de logement à l'axe 6 précédent.

Conformément à l'invention, deux flasques latéraux 2 qui seront également vus plus facilement sur la figure 2 suivante, sont munis d'une découpure carrée et d'ergots d'extrémité destinés à être insérés dans des encoches de la chape femelle. Les découpures des flasques latéraux 2 sont destinées à recevoir des joues 5 de maintien en place de la bague 4.

Comme il a déjà été expliqué précédemment, les chapes femelle 1 et mâle 3, ainsi que les flasques latéraux 2 sont réalisés en alliage de titane et de façon préférentielle et plus précise encore dans l'alliage TA6V. Au contraire, la bague 4, les joues 5 et l'axe 6 sont réalisés en pyrocarbone pour les propriétés de résistance au frottement de ce dernier matériau. De plus, et ceci est une particularité importante de l'invention, la bague 4 qui est montée serrée dans l'orifice de la partie femelle mâle de la chape mâle 3 a une longueur légèrement supérieure à celle de l'orifice précédent de façon à émerger de chaque côté de celui-ci. Par ailleurs, et ceci est une deuxième caractéristique importante de la structure de la prothèse, objet de l'invention, l'épaisseur des joues 5 est supérieure à celle des flasques latéraux 2 dans lesquels elles sont insérées. Les deux conditions précédentes combinées conduisent alors au résultat essentiel de l'invention, selon lequel lors de la rotation de l'articulation, les seules pièces qui sont en contact et qui par conséquent développent des frottements les unes contre les autres, sont des pièces en pyrocarbone à l'exclusion de toute pièce en alliage de titane. En effet, ce sont les joues 5 qui viennent en contact de la bague 4 et cette dernière est elle-même montée rotative autour de l'axe 6. De cette façon, la solidité et la longévité de la prothèse articulaire, objet de l'invention sont complètement assurées.

Sur la figure 2, on retrouve les éléments constitutifs de la prothèse articulaire pivotante sous une forme démontée qui permet effectivement de mieux apercevoir les formes exactes de chaque pièce, les orifices des chapes femelle 1 et mâle 3, ainsi que celles des flasques latéraux 2 dont l'ajourement carré central est destiné à contenir le profil également carré des joues latérales 5. La bague 4 et l'axe 6 ont des dimensions respectives leur permettant de s'emboîter l'une dans l'autre, l'ensemble étant alors monté et fixé par engagement en force de l'axe 6 dans les orifices de la chape femelle 1. On voit également sur les flasques latéraux 2, les ergots d'extrémité 8 qui permettent leur fixation dans des encoches 10 prévues à cet effet à la base de la chape femelle 1.

En fin de montage il peut être avantageux d'effectuer un point de soudure par exemple avec un laser pour solidariser définitivement les flasques latéraux 2 et la chape femelle 1.

Un essai de résistance à l'usure d'une prothèse articulaire selon l'invention a été effectuée sur une machine d'essai simulant le mouvement du doigt en flexion-extension avec un débattement angulaire de 120° et une cadence de deux cycles par seconde. Des prothèses soumises à ce test, puis démontées et examinées n'ont montré aucune trace d'usure observable à la loupe binoculaire au grandissement 50 après 20 millions de cycles.

Enfin, la présente invention n'est pas limitée à l'utilisation de pyrocarbone pour la bague 4, les joues 5 et l'axe 6 ; les matériaux biocompatibles et possédant un coefficient de frottement faible peuvent également être employés.

De même, le titane ou l'un de ses alliages peut être remplacé par tout autre matériau biocompatible et possédant des propriétés mécaniques suffisantes pour être utilisé dans une prothèse articulaire selon l'invention.

## Revendications

1. Prothèse articulaire digitale pour articulations métacarpophalangiennes et interphalangiennes comprenant deux pièces diaphysaires, (1,3) implantables dans les os des phalanges et/ou du métacarpe, solidaires d'une articulation pivotante comprenant :
- une chape femelle (1) solidaire de la première pièce diaphysaire (1), munie de deux orifices alignés et transverses par rapport à cette première pièce ;
- une chape mâle (3), munie d'un orifice transverse par rapport à la deuxième pièce diaphysaire, coopérant avec la chape femelle et solidaire de la deuxième pièce diaphysaire ;
- un axe (6), monté à force dans les deux orifices de la chape femelle (1) et s'étendant au travers de l'orifice de la chape mâle (3) ;
- une bague (4) entourant l'axe (6), engagée en force dans l'orifice de la chape mâle (3) et émergeant de chaque côté de cet orifice ;
caractérisée en ce que l'articulation comprend en outre deux flasques latéraux (2), munis d'une découpure carrée et d'ergots d'extrémités (8) insérables dans des encoches (10) de la chape femelle, ces découpures étant destinées à recevoir des joues (5) de maintien en place de la bague, l'épaisseur de chaque joue (5) étant supérieure à celle des flasques latéraux (2).

2. Prothèse articulaire selon la revendication 1, caractérisée en ce que les chapes mâle (3) et femelle (1) ainsi que les flasques latéraux (2) sont en alliage de titane et la bague (4), l'axe (6) et les joues (5) sont en pyrocarbone de façon à ce que les frottements lors de la rotation de l'articulation aient lieu uniquement entre des pièces en pyrocarbone.

## Claims

1. Finger joint prosthesis for metacarpophalangeal and interphalangeal joints having two diaphysed parts (1, 3) implantable in phalangeal and/or metacarpeal bones, integral with a pivoting joint, characterized in that said joint comprises:
- a female cap (1) joined to the first diaphysed part (1), provided with two aligned openings transverse with respect to said first part.
- a male cap (3), provided with a transverse opening with respect to the second diaphysed part and cooperating with the female cap and integral with the second diaphysed part,
- a shaft (6), force fitted into the two openings of the female cap (1) and extending through the opening of the male cap (3),
- a collar (4) surrounding the shaft (6) and force fitted in the opening of the male cap (3) and emerging from each side of said opening,
characterized in that the joint also comprises two lateral flanges (2), each provided with a square cutout and end lugs (8) insertable into notches (10) of the female cap, said cutouts serving to receive disks (5) for holding in place the collar, the thickness of each disk (5) exceeding that of the lateral flanges (2).

2. Joint prosthesis according to claim 1, characterized in that the male (3) and female (1) caps, as well as the lateral flanges (2) are made from titanium alloy and the collar (4), the shaft (6) and the disks (5) are made from pyrocarbon, so that the friction taking place during the rotation of the joint only occurs between the pyrocarbon parts.

## Patentansprüche

1. Fingergelenkprothese für metakarpale und interphalangäre Gelenke, zwei Diaphyse- bzw. diaphysäre Teile (1,3) enthaltend, implantierbar in die Fingerglieder- und/oder Mittelhandknochen, verbunden mit einem schwenkbaren Gelenk, umfassend:
- eine Gelenkgabel (1), ein Stück bildend mit dem ersten Diaphyse-Teil (1), versehen mit zwei fluchtenden, bezüglich dieses ersten Teils quergerichteten Öffnungen;
- einen Gelenkkopf (3), versehen mit einer bezüglich des zweiten Diaphyse-Teils quergerichteten Öffnung, zusammenwirkend mit der Gelenkgabel und ein Stück bildend mit dem zweiten Diaphyse-Teil;
- eine mit Kraftaufwand in die beiden Öffnungen der Gelenkgabel (1) eingetriebene Achse (6), die sich durch die Öffnung des Gelenkkopfes (3) hindurch erstreckt;
- einen Ring (4), der die Achse (6) umgibt, mit Kraftaufwand eingepreßt in die Öffnung des Gelenkkopfes (3) und auf jeder Seite dieser Öffnung herausstehend;
**dadurch gekennzeichnet**,
daß das Gelenk außerdem zwei seitliche Scheiben (2) umfaßt, versehen mit einer quadratischen Durchbrechung und Vorsprüngen (8), die sich einsetzen lassen in Kerben (10) der Gelenkgabel, wobei diese Durchbrechungen dazu bestimmt sind, Arretierstücke (5) für das Instellunghalten des Rings aufzunehmen und die Dicke jedes Arretierstücks (5) größer ist als die der seitlichen Scheiben (2).

2. Gelenkprothese nach Anspruch 1, dadurch gekennzeichnet, daß der Gelenkkopf (3) und die Gelenkgabel (1) sowie die seitlichen Scheiben (2) aus Titanlegierung sind, und der Ring (4), die Achse (6) und die Arretierstücke (5) aus Pyrocarbon sind, so daß die Reibungen bei der Rotation des Gelenks nur zwischen den Teilen aus Pyrocarbon stattfinden.
